# EUROPEAN PATENT APPLICATION

(11) **EP 4 063 396 A1**
(43) Date of publication of application: **28.09.2022**
(21) Application number: 20888942.8
(22) Date of filing: 18.11.2020
(51) Int. Cl.: C07K 16/40, C12N 15/13, A61K 39/395, A61K 49/00, A61K 51/10, A61P 9/12, A61P 3/06, A61P 9/00

(54) **ANTI-PCSK9 ANTIBODY AND USE THEREOF**

(30) Priority: 18.11.2019 CN 201911132974; 18.11.2019 CN 201911133187
(71) Applicant: AD Pharmaceutical Co., Ltd., Guangdong 510530 (CN)
(72) Inventor: LI, Baiyong, Zhongshan Guangdong 528437 (CN); XIA, Yu, Zhongshan Guangdong 528437 (CN); WANG, Zhongmin, Zhongshan Guangdong 528437 (CN); JIN, Xiaoping, Zhongshan Guangdong 528437 (CN)
(74) Representative: Cooley (UK) LLP
(86) International application number: PCT/CN2020/129763
(87) International publication number: WO 2021/098720

(57) **Abstract**

Provided are an anti-PCSK9 antibody and a use thereof preferably for the treatment of hypercholesterolemia.

SEQUENCE LISTING

<110>  AD Pharmaceuticals Co., Ltd.

<120>  ANTI-PCSK9 ANTIBODY AND USE THEREOF

<130>  IP190579

<160>  12

<170>  PatentIn version 3.3

<210>  1
<211>  369
<212>  DNA
<213>  Artificial sequences

<400>  1
gaggtgcagc tggtggagtc tggaggaggc ctggtgcagc ccggaagatc tctgagactg      60

agttgcgccg cttcaggatt cacctttagc tcctacagca tgaactgggt gcggcaggct     120

cctggcaagg ggctggagtg ggtctccgga atctctagtt caagctccta cattagctat     180

gcagactccg tccagggaag gttcaccatc tctcgcgata acggcaagaa cagcctgtat     240

ctgcagatga acagcctgcg agcagaggac acagccctgt acttctgtgc cagagaatat     300

gacttctggt ccgcctatta cgacgccttc gatgtctggg gacaggggac tatggtcact     360

gtctcaagc                                                             369


<210>  2
<211>  123
<212>  PRT
<213>  Artificial sequences

<400>  2

Glu Val Gln Leu Val Glu Ser Gly Gly Gly Leu Val Gln Pro Gly Arg
1               5                   10                  15


Ser Leu Arg Leu Ser Cys Ala Ala Ser Gly Phe Thr Phe Ser Ser Tyr
            20                  25                  30


Ser Met Asn Trp Val Arg Gln Ala Pro Gly Lys Gly Leu Glu Trp Val
        35                  40                  45


Ser Gly Ile Ser Ser Ser Ser Ser Tyr Ile Ser Tyr Ala Asp Ser Val
    50                  55                  60


Gln Gly Arg Phe Thr Ile Ser Arg Asp Asn Gly Lys Asn Ser Leu Tyr
65                  70                  75                  80


Leu Gln Met Asn Ser Leu Arg Ala Glu Asp Thr Ala Leu Tyr Phe Cys
                85                  90                  95

Ala Arg Glu Tyr Asp Phe Trp Ser Ala Tyr Tyr Asp Ala Phe Asp Val
            100                 105                 110

Trp Gly Gln Gly Thr Met Val Thr Val Ser Ser
        115                 120

<210> 3
<211> 333
<212> DNA
<213> Artificial sequences

<400> 3
cagagcgaac tgactcagcc aagaagcgtc agtggatcac ctggccagag cgtgacaatc 60

tcctgcaccg gcacaagcag gaacattggc gggggaaatg acgtccactg gtaccagcag 120

catccaggga aggcccccaa actgctgatc tccggagtga ttgagcggag ctccggcgtc 180

cccgatagat tcagcgggtc caagtctgga aacacagctt ctctgactat cagtggcctg 240

caggcagagg acgaagccga ttactattgc cagtctttcg acggcagtct gtcagggagc 300

gtgtttggca ctgggaccga tgtgaccgtc ctg 333

<210> 4
<211> 111
<212> PRT
<213> Artificial sequences

<400> 4

Gln Ser Glu Leu Thr Gln Pro Arg Ser Val Ser Gly Ser Pro Gly Gln
1               5                   10                  15

Ser Val Thr Ile Ser Cys Thr Gly Thr Ser Arg Asn Ile Gly Gly Gly
            20                  25                  30

Asn Asp Val His Trp Tyr Gln Gln His Pro Gly Lys Ala Pro Lys Leu
        35                  40                  45

Leu Ile Ser Gly Val Ile Glu Arg Ser Ser Gly Val Pro Asp Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Gln Ser Phe Asp Gly Ser
                85                  90                  95

Leu Ser Gly Ser Val Phe Gly Thr Gly Thr Asp Val Thr Val Leu
            100                 105                 110

<210>  5
<211>  8
<212>  PRT
<213>  Artificial sequences

<400>  5

Gly Phe Thr Phe Ser Ser Tyr Ser
1               5


<210>  6
<211>  8
<212>  PRT
<213> Artificial sequences

<400>  6

Ile Ser Ser Ser Ser Ser Tyr Ile
1               5


<210>  7
<211>  14
<212>  PRT
<213>  Artificial sequences

<400>  7

Glu Tyr Asp Phe Trp Ser Ala Tyr Tyr Asp Ala Phe Asp Val
1               5                   10


<210>  8
<211>  9
<212>  PRT
<213>  Artificial sequences

<400>  8

Ser Arg Asn Ile Gly Gly Gly Asn Asp
1               5


<210>  9
<211>  3
<212>  PRT
<213>  Artificial sequences

<400>  9

Gly Val Ile
1


<210>  10
<211>  11
<212>  PRT
<213>  Artificial sequences

<400>  10

Gln Ser Phe Asp Gly Ser Leu Ser Gly Ser Val

1               5                   10


<210>  11
<211>  443
<212>  PRT
<213>  Artificial sequences

<400>  11

Glu Val Gln Leu Glu Gln Ser Gly Ala Glu Leu Met Lys Pro Gly Ala
1               5                   10                  15


Ser Val Lys Ile Ser Cys Lys Ala Thr Gly Tyr Thr Phe Thr Thr Tyr
            20                  25                  30


Trp Ile Glu Trp Ile Lys Gln Arg Pro Gly His Ser Leu Glu Trp Ile
        35                  40                  45


Gly Glu Ile Leu Pro Gly Ser Asp Ser Thr Tyr Tyr Asn Glu Lys Val
    50                  55                  60


Lys Gly Lys Val Thr Phe Thr Ala Asp Ala Ser Ser Asn Thr Ala Tyr
65                  70                  75                  80


Met Gln Leu Ser Ser Leu Thr Ser Glu Asp Ser Ala Val Tyr Tyr Cys
                85                  90                  95


Ala Arg Gly Asp Gly Phe Tyr Val Tyr Trp Gly Gln Gly Thr Thr Leu
            100                 105                 110


Thr Val Ser Ser Ala Ser Thr Lys Gly Pro Ser Val Phe Pro Leu Ala
        115                 120                 125


Pro Cys Ser Arg Ser Thr Ser Glu Ser Thr Ala Ala Leu Gly Cys Leu
    130                 135                 140


Val Lys Asp Tyr Phe Pro Glu Pro Val Thr Val Ser Trp Asn Ser Gly
145                 150                 155                 160


Ala Leu Thr Ser Gly Val His Thr Phe Pro Ala Val Leu Gln Ser Ser
                165                 170                 175


Gly Leu Tyr Ser Leu Ser Ser Val Val Thr Val Pro Ser Ser Ser Leu
            180                 185                 190


Gly Thr Lys Thr Tyr Thr Cys Asn Val Asp His Lys Pro Ser Asn Thr
        195                 200                 205


Lys Val Asp Lys Arg Val Glu Ser Lys Tyr Gly Pro Pro Cys Pro Pro

210                 215                 220

Cys Pro Ala Pro Glu Phe Leu Gly Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser Gln Glu Asp Pro Glu Val Gln Phe Asn
            260                 265                 270

Trp Tyr Val Asp Gly Val Glu Val His Asn Ala Lys Thr Lys Pro Arg
        275                 280                 285

Glu Glu Gln Phe Asn Ser Thr Tyr Arg Val Val Ser Val Leu Thr Val
    290                 295                 300

Leu His Gln Asp Trp Leu Asn Gly Lys Glu Tyr Lys Cys Lys Val Ser
305                 310                 315                 320

Asn Lys Gly Leu Pro Ser Ser Ile Glu Lys Thr Ile Ser Lys Ala Lys
                325                 330                 335

Gly Gln Pro Arg Glu Pro Gln Val Tyr Thr Leu Pro Pro Ser Gln Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Val Leu Asp Ser Asp Gly Ser Phe
385                 390                 395                 400

Phe Leu Tyr Ser Arg Leu Thr Val Asp Lys Ser Arg Trp Gln Glu Gly
                405                 410                 415

Asn Val Phe Ser Cys Ser Val Met His Glu Ala Leu His Asn His Tyr
            420                 425                 430

Thr Gln Lys Ser Leu Ser Leu Ser Leu Gly Lys
        435                 440

<210>  12
<211>  214
<212>  PRT
<213>  Artificial sequences

<400>  12

Asp Ile Glu Leu Thr Gln Ser Pro Ala Thr Leu Ser Val Thr Pro Gly
1               5                   10                  15

Asp Ser Val Ser Leu Ser Cys Arg Ala Ser Gln Ser Ile Ser Asn Asn
            20                  25                  30

Leu His Trp Tyr Gln Gln Lys Ser His Glu Ser Pro Arg Leu Leu Ile
        35                  40                  45

Lys Tyr Thr Ser Gln Ser Met Ser Gly Ile Pro Ser Arg Phe Ser Gly
    50                  55                  60

Ser Gly Ser Gly Thr Asp Phe Thr Leu Ser Ile Asn Ser Val Glu Thr
65                  70                  75                  80

Glu Asp Phe Gly Val Tyr Phe Cys Gln Gln Ser Gly Ser Trp Pro Arg
                85                  90                  95

Thr Phe Gly Gly Gly Thr Lys Leu Asp Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

## Description

### TECHNICAL FIELD

The present application relates to the field of medical technology and particularly, to the treatment of hypercholesterolemia. More particularly, the present application relates to use of anti-PCSK9 antibodies in treating hypercholesterolemia.

### BACKGROUND

PCSK9 is a secreted serine protease that binds to and degrades low-density lipoprotein receptor (LDLR) on cell surface, resulting in a reduced level of LDLR on the surface of cell membrane. PCSK9 is prominently synthesized and secreted into the blood by the liver. LDLR is widely distributed on the surface of liver cell membrane and liver cell lines such as HepG2, Hep3B. Inhibiting the binding of PCSK9 to LDLR can maintain the level of LDLR on the surface of cell membrane, thereby mediating the entry of plasma cholesterol LDL to cells and maintaining the plasma cholesterol level. Hypercholesterolemia is a disease characterized by defected lipid metabolism, of which some patients may carry an autosomal dominant genetic mutation called familial hypercholesterolemia (FH), mainly manifested by a significant increase in plasma low-density lipoprotein cholesterol (LDL-C) level. FH can be classified into 4 types, heterozygosity, homozygosity, compound heterozygosity and double heterozygosity, among which the first two constitute the majority. Homozygous familial hypercholesterolemia (HoFH) is relatively rare, with a prevalence of 1 to 3 per million. HoFH disease is characterized by elevated LDL-C level and increased risk of early atherosclerotic cardiovascular disease. The European Atherosclerosis Society (EAS) states that HoFH patients typically develop atherosclerotic cardiovascular disease around age of 20 and die around age of 30 without intervention. The prominent therapies for HoFH include therapeutic lifestyle changes, medications, and other treatments such as plasma lipoprotein exchange. Statins are still the prominent medications. However, for HoFH patients, the high blood lipid level often requires combinations of statins and ezetimibe combination therapy.

The prevalence of heterozygous familial hypercholesterolemia (HeFH) is about 1:500 to 1:200, which means about 14 to 34 million HeFH patients around the world. The prominent therapies for HeFH include therapeutic lifestyle changes, medications, and other treatments such as plasma lipoprotein exchange. Statins are still the prominent medications. However, for HeFH patients, the high blood lipid level often requires a combination therapy of statins and ezetimibe. PCSK9 inhibitors can directly prevent circulating PCSK9 from binding to LDLR, reduce PCSK9-mediated degradation of LDLR, enhance the cleaning capacity of LDLR-C, and further reduce the blood lipid level in the patients on the basis of basic lipid-lowering agents. There are two monoclonal antibodies targeting PCSK9 in the market: alirocumab and evolocumab. Alirocumab (formerly REGN727/SAR236553), a fully human anti-PCSK9 monoclonal antibody developed by Regeneron Pharmaceuticals, U.S.A., was approved by the United States Food and Drug Administration (FDA) in 2015 as a second-line therapy to reduce LDL-C, and is indicated for adult patients with hereditary hypercholesterolemia and adult patients with atherosclerosis who have failed dietary intervention and treatment with statins and require LDL-C reduction. On Aug. 27, 2015, the US FDA approved use of evolocumab injection in combination with dietary control and a maximum tolerated dose of statins in patients with HoFH and adult patients with clinical atherosclerotic cardiovascular diseases. On Jul. 31, 2018, the National Medical Product Administration (NMPA), PRC approved evolocumab injection for treatment of HoFH (i.e. homozygous familial hypercholesterolemia) in adults or juveniles aged 12 or above.

Cardiovascular diseases are one of the main risk factors of death in patients with hyperlipidemia, and the current research results suggest that potent hypolipidemia therapies, such as statins, can significantly reduce the cardiovascular disease attack and death risk in patients with hyperlipidemia. Anti-PCSK9 antibodies are potent lipid-lowering agents, of which the efficacy in preventing cardiovascular disease attack and reducing the death risk in patients with hyperlipidemia has also been demonstrated. The clinical research results of anti-PCSK9 antibody evolocumab demonstrated that receiving evolocumab treatment can significantly reduce the risk of cardiovascular disease attack in patients with hyperlipidemia, and compared with the placebo control group, the incidences of myocardial infarction, stroke and conditions requiring coronary revascularization procedure are significantly reduced in the patients receiving evolocumab treatment. Large-scale clinical studies of another marketed anti-PCSK9 antibody, alirocumab, also demonstrated a protective effect on the cardiovascular system. Compared with the placebo control, the anti-PCSK9 antibody can reduce the overall risk of developing major adverse cardiovascular events, including acute myocardial infarction, ischemic stroke, death of coronary heart disease (CHD), or unstable angina requiring hospitalization, by 15%. Based on the results of these clinical studies, the two anti-PCSK9 antibodies were approved by the FDA in 2017 and 2019, respectively, for preventing heart disease and stroke in adult patients with cardiovascular disease.

### SUMMARY

One aspect of the present application provides an inhibitor of the interaction between PCSK9 and LDLR, preferably an anti-PCSK9 antibody or an antigen-binding fragment thereof, for treating hypercholesterolemia (more preferably homozygous familial hypercholesterolemia or "HoFH", or heterozygous familial hypercholesterolemia or "HeFH", or hypercholesterolemia in a patient with extremely high or high risk of cardiovascular disease).

In some specific embodiments, the antibody or the antigen-binding fragment thereof is a monoclonal antibody or an antigen-binding fragment thereof that specifically binds to human PCSK9 and blocks the binding of human PCSK9 to LDLR.

In some embodiments, the anti-PCSK9 antibody is a humanized antibody.

In some embodiments, the anti-PCSK9 antibody comprises:
a heavy chain variable region comprising an amino acid sequence set forth in SEQ ID NO: 2 or an amino acid sequence having at least 80% homology, preferably at least 85% homology, more preferably at least 90%, 91%, 92%, 93%, 94%, 95%, 96%, 97%, 98% or 99% homology thereto; and
a light chain variable region comprising an amino acid sequence set forth in SEQ ID NO: 4 or an amino acid sequence having at least 80% homology, preferably at least 85% homology, more preferably at least 90%, 91%, 92%, 93%, 94%, 95%, 96%, 97%, 98% or 99% homology thereto.

In some embodiments, the CDR sequences of the antibody are as follows:
HCDR1: GFTFSSYS (SEQ ID NO: 5);
HCDR2: ISSSSSYI (SEQ ID NO: 6);
HCDR3: EYDFWSAYYDAFDV (SEQ ID NO: 7);
LCDR1: SRNIGGGND (SEQ ID NO: 8);
LCDR2: GVI (SEQ ID NO: 9); and
LCDR3: QSFDGSLSGSV (SEQ ID NO: 10).

Another aspect of the present application provides a pharmaceutical composition, preferably for treating hypercholesterolemia (more preferably HoFH, HeFH or hypercholesterolemia in a patient with extremely high or high risk of cardiovascular disease), comprising: an inhibitor of the interaction between PCSK9 and LDLR (and a statin), and a pharmaceutically acceptable carrier.

In some embodiments, the inhibitor of the interaction between PCSK9 and LDLR is in a form suitable for a parenteral or non-parenteral route of administration.

Another aspect of the present application provides a kit, preferably for treating hypercholesterolemia (more preferably HoFH, HeFH or hypercholesterolemia in a patient with extremely high or high risk of cardiovascular disease), comprising the anti-PCSK9 antibody or the antigen-binding fragment thereof disclosed herein. In some embodiments, the kit further comprises a statin, and/or ezetimibe.

The present application is further intended to at least provide a method for treating hypercholesterolemia (more preferably HoFH, HeFH or hypercholesterolemia in a patient with extremely high or high risk of cardiovascular disease), comprising: administering (e.g., parenterally or non-parenterally) to a subject in need a therapeutically effective amount of the inhibitor of the interaction between PCSK9 and LDLR disclosed herein (preferably the anti-PCSK9 antibody or the antigen-binding fragment thereof). Preferably, a statin is administered to the subject in combination. More preferably, a statin and ezetimibe are administered to the subject in combination. In some embodiments, the inhibitor, the statin and/or ezetimibe are administered simultaneously or separately.

In some embodiments, the statin is a 3-hydroxy-3-methylglutaryl coenzyme A (HMG-CoA) reductase inhibitor.

In some embodiments, the statin, i.e., the 3-hydroxy-3-methylglutaryl coenzyme A (HMG-CoA) reductase inhibitor, includes lovastatin, pravastatin, simvastatin, fluvastatin, atorvastatin, rosuvastatin and pitavastatin or any combination thereof, etc.

In certain embodiments, the hypercholesterolemia (more preferably HoFH, HeFH or hypercholesterolemia in a patient with extremely high or high risk of cardiovascular disease) is caused by 1 (pair of) or more (pairs of) allelic or non-allelic gene mutations in LDLR, apolipoprotein B (ApoB), PCSK9 or low-density lipoprotein receptor adaptor protein 1 (LDLRAP1) genes, etc., wherein the allelic gene mutation includes LDLR dysfunctional mutation, ApoB gene mutation affecting the binding of LDL to LDLR, functional mutation resulting in increased affinity of PCSK9 for LDLR, LDLRAP1 dysfunctional mutation resulting in decreased LDL internalization activity, etc. Variants known to cause FH in the LDLR, APOB or PCSK9 genes are described, for example, in Canadian Journal of Cardiology, 34, (2018), 1553-1563, Canadian Cardiovascular Society Position Statement on Familial Hypercholesterolemia: Update 2018.

In certain embodiments, the hypercholesterolemia (more preferably HoFH, HeFH or hypercholesterolemia in a patient with extremely high or high risk of cardiovascular disease) exhibits LDL-C > 13 mmol/L (500 mg/dL) when previously untreated or LDL-C ≥ 8 mmol/L (300 mg/dL) when previously treated (with a high dose/maximum tolerated dose of a statin in combination with ezetimibe), combined with skin or tendon xanthoma before age of 10 or both parents carrying 1 (pair of) or more (pairs of) allelic gene mutations in LDLR, ApoB, PCSK9 or LDLRAP1 genes, etc.

In certain embodiments, the hypercholesterolemia (more preferably HeFH) satisfies the following a) and b): a) the hypercholesterolemia is caused by 1 (pair of) or more (pairs of) unpaired allelic gene mutations in LDLR, apolipoprotein B (ApoB), PCSK9 or low-density lipoprotein receptor adaptor protein 1 (LDLRAP1) genes, etc., as described above, b) the patient has a serum LDL-C level ≥ 4.7 mmol/L (180 mg/dL) when previously untreated with a lipid regulating drug; or satisfies at least 1 of the following c) and d) simultaneously: c) a patient having skin or tendon xanthoma or a patient aged < 45 years with arcus lipoides corneae, and d) having a first-degree relative with FH or early adult atherosclerotic cardiovascular disease (ASCVD), particularly coronary heart disease.

The present application is further intended to at least provide use of the inhibitor of the interaction between PCSK9 and LDLR disclosed herein (preferably the anti-PCSK9 antibody or the antigen-binding fragment thereof) in preparing a medicament for treating hypercholesterolemia (more preferably HoFH, HeFH or hypercholesterolemia in a patient with extremely high or high risk of cardiovascular disease).

The present application further provides a method for treating a patient with hypercholesterolemia (more preferably HoFH, HeFH or hypercholesterolemia in a patient with extremely high or high risk of cardiovascular disease), comprising: (i) genotyping the patient, (ii) measuring LDL-C level in a sample from the patient, and (iii) administering to the patient, when the patient is identified with homozygous (or heterozygous) familial hypercholesterolemia, a therapeutically effective amount of the anti-PCSK9 antibody or the antigen-binding portion thereof disclosed herein, preferably in combination with a statin, more preferably in combination with a statin and ezetimibe.

In some embodiments, the sample is serum, plasma or whole blood.

In certain embodiments of the present invention, the hypercholesterolemia in the patient with extremely high or high risk of cardiovascular disease refers to simultaneously satisfying the following condition 1 and condition 2, wherein the condition 1 is satisfying any of a) to h) (refer to Guidelines for Prevention and Treatment of Dyslipidemia in Chinese Adults (2016 Edition): a) a history of coronary artery disease (e.g. acute coronary syndrome, stable coronary heart disease, post-revascularization, ischemic cardiomyopathy), b) a history of ischemic stroke (excluding lacunar infarction), c) transient ischemic attack, d) peripheral atherosclerosis (e.g. carotid atherosclerosis, post-carotid endarterectomy, post-carotid stent placement, post-revascularization, lower limb atherosclerotic disease, post-lower limb artery revascularization), e) LDL-C ≥ 4.9 mmol/L (190 mg/dL) or TC ≥ 7.2 mmol/L, f) type 2 diabetes patients aged 40 years and above with 1.8 mmol/L (70 mg/dL) ≤ LDL-C < 4.9 mmol/L (190 mg/dL), g) chronic renal disease in stage 3 (i.e., with measured and calculated glomerular filtration rate 30 mL/min/1.73 m² ≤ eGFR < 60 mL/min/1.73 m²), h) at least 3 of the following risk factors: male aged ≥ 45 and female aged ≥ 55; hypertension; smoking; a family history of CVD (for first-degree relatives: male aged < 55 and female aged < 65); HDL cholesterol < 40 mg/dL; obesity (BMI ≥ 28 kg/m²); and the condition 2 is satisfying either a) or b): after 4 weeks of stable basal lipid-lowering agent treatment: a) LDL-C level > 1.8 mmol/L (70 mg/dL) for patients with extremely high risk, or b) LDL-C level > 2.6 mmol/L (100 mg/dL) for patients with high risk.

The present application is further intended to at least provide a method for preparing a pharmaceutical formulation (pharmaceutical composition) of an inhibitor of the interaction between PCSK9 and LDLR (preferably an anti-PCSK9 antibody or an antigen-binding fragment thereof) for treating hypercholesterolemia (preferably HoFH, HeFH or hypercholesterolemia in a patient with extremely high or high risk of cardiovascular disease).

In some embodiments, the anti-PCSK9 antibody is administered in a cycle of 2 weeks (14 days) or 4 weeks (28 days), and preferably, subcutaneously administered on the first day (D1) of each cycle. That is, the anti-PCSK9 antibody is administered once every two weeks (q2w) or four weeks (q4w).

In some embodiments, related is a single dose unit, preferably for treating familial hypercholesterolemia (more preferably HoFH, HeFH or hypercholesterolemia in a patient with extremely high or high risk of cardiovascular disease), comprising: 0.1-60 mg of the anti-PCSK9 antibody or the antigen-binding fragment thereof disclosed herein.

Another aspect of the present invention relates to use of 0.1-60 mg of the anti-PCSK9 antibody or the antigen-binding fragment thereof disclosed herein in preparing a single dose unit for treating familial hypercholesterolemia (more preferably HoFH, HeFH or hypercholesterolemia in a patient with extremely high or high risk of cardiovascular disease).

### MAB1

As used herein, MAB1 is an anti-PCSK9 monoclonal antibody, the sequence and structure of which can be found in reference Patent No. WO2016/127912A1. In the monoclonal antibody MAB1, the HCDR1 comprises a sequence GFTFSSYS (SEQ ID NO: 5), the HCDR2 comprises a sequence ISSSSSYI (SEQ ID NO: 6), the HCDR3 comprises a sequence EYDFWSAYYDAFDV (SEQ ID NO: 7), the LCDR1 comprises a sequence SRNIGGGND (SEQ ID NO: 8), the LCDR2 comprises a sequence GVI (SEQ ID NO: 9), and the LCDR3 comprises a sequence QSFDGSLSGSV (SEQ ID NO: 10).

### Definitions and Descriptions

The following terms used herein have the following meanings, unless otherwise specified. A certain term, unless otherwise specifically defined, should not be considered uncertain or unclear, but construed according to its common meaning in the field. When referring to a trade name, it is intended to refer to its corresponding commercial product, composition or its active ingredient.

As used herein, the term "antibody" refers to an antigen-binding protein having at least one antigen-binding domain. The antibody and the fragment thereof disclosed herein can be the whole antibody or any fragment thereof. Thus, the antibody and the fragment thereof disclosed herein include a monoclonal antibody or a fragment thereof and an antibody variant or a fragment thereof, as well as an immunoconjugate. The antibody and the fragment thereof may also include a recombinant polypeptide, a fusion protein, and a bispecific antibody. The anti-PCSK9 antibody and the fragment thereof disclosed herein can be of IgG1, IgG2, IgG3, or IgG4 isotype.

The term "isotype" refers to the class of antibodies encoded by the heavy chain constant region gene. In one embodiment, the anti-PCSK9 antibody and the fragment thereof disclosed herein are of the IgG1 or IgG4 isotype. The anti-PCSK9 antibody and the fragment thereof disclosed herein can be derived from any species, including but not limited to mouse, rat, rabbit, primate, llama, and human. The anti-PCSK9 antibody and the fragment thereof may be a chimeric antibody, a humanized antibody or an intact human antibody.

The term "humanized antibody" refers to an antibody in which the antigen-binding site is derived from a non-human species and the variable region framework is derived from human immunoglobulin sequences. The humanized antibody may comprise substitutions in the framework regions such that the framework may not be an exact copy of the expressed human immunoglobulin or germline gene sequence.

The term "gene mutation" refers to a sudden, heritable variation occurring in a genomic DNA molecule. In a molecular level, a gene mutation is a structural change in the base pair composition or arrangement of a gene. It includes base substitution mutation, frameshift mutation, deletion mutation and insertion mutation.

The term "isolated antibody" refers to an antibody that is substantially free of other antibodies having different antigenic specificities (e.g., an isolated antibody that specifically binds to PCSK9 is substantially free of antibodies that specifically bind to antigens apart from PCSK9). However, an isolated antibody that specifically binds to PCSK9 may have cross-reactivity with other antigens (such as PCSK9 molecules from different species). Furthermore, the isolated antibody may be substantially free of other cellular materials and/or chemicals.

The term "monoclonal antibody" ("mAb") refers to an antibody molecule of a single molecule composition. A monoclonal antibody composition exhibits a single binding specificity and affinity for a particular epitope or, in the case of bispecific monoclonal antibody, a dual binding specificity for two different epitopes. mAb is an example of the isolated antibody. mAbs can be produced by hybridoma techniques, recombinant techniques, transgenic techniques, or other techniques known to those skilled in the art.

An "antigen-binding portion" (also referred to as an "antigen-binding fragment") of an antibody refers to one or more fragments of the antibody that retain the ability to specifically bind to an antigen to which an intact antibody binds. Examples of antigen-binding fragments include Fab fragments, Fab' fragments, F(ab)' fragments, Fv fragments, isolated CDR regions, single chain Fv molecules (scFv), F(ab')₂, Fd, dAb, Fab/c, bivalent antibodies and domain antibodies.

As used herein, the term "derived", when used to refer to a molecule or polypeptide relative to a reference antibody or other binding proteins, means a molecule or polypeptide that is capable of specifically binding to the same epitope as the reference antibody or other binding proteins.

As used herein, the term "EC₅₀" refers to an effective concentration causing 50% of the maximal response of an antibody. As used herein, the term "IC₅₀" refers to an inhibitory concentration causing 50% of the maximal response of an antibody. Both EC₅₀ and IC₅₀ can be measured by ELISA or FACS assay or any other method known in the art.

The term "treatment" or "treating" usually refers to operations for acquiring needed pharmacological effect and/or physiological effect. In terms of fully or partially preventing a disease or a symptom thereof, the effect can be preventive; and/or in terms of partially or fully stabilizing or curing the disease and/or a side effect of the disease, the effect can be therapeutic. As used herein, "treatment" or "treating" covers any treatment to a disease in a patient, including (a) preventing a disease or a symptom in a patient susceptible to the disease or the symptom that has not yet been diagnosed; (b) inhibiting a symptom of a disease, i.e., arresting its development; or (c) relieving a symptom of a disease, i.e., causing regression of the disease or the symptom. As used herein, the term "general treatment" refers to treatment in which a drug substance is transported through the bloodstream to reach and affect cells of the whole body.

As used herein, the term "subject" refers to a mammal, such as a rodent, feline, canine, and primate. Preferably, the subject according to the present application is a human.

"Administer", "administration" or "administering" means physically introducing a composition comprising a therapeutic agent to a subject using any of a variety of methods and delivery systems known to those skilled in the art. Routes of administration of immune checkpoint inhibitors (e.g., an anti-PCSK9 antibody) include intravenous, intramuscular, subcutaneous, intraperitoneal, spinal, or other parenteral routes of administration, for example, by injection or infusion. As used herein, the phrase "parenteral administration" refers to modes of administration except for enteral and local administration, typically by injection, including but not limited to, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and *in vivo* electroporation. In certain embodiments, the immune checkpoint inhibitor (e.g., an anti-PCSK9 antibody) is administered by a non-parenteral route, and in certain embodiments, it is administered orally. Other non-parenteral routes include local, epidermal or mucosal routes of administration, for example, intranasal, vaginal, rectal, sublingual or local administration. Administration may also be performed, e.g., once, multiple times, and/or over one or more extended periods of time.

As used herein, an "adverse event" (AE) is any adverse and often unintended or undesirable sign (including abnormal laboratory findings), symptom, or disease associated with the use of medical therapy. For example, an adverse event may be associated with a liver secreting PCSK9 in response to a therapy. The medical treatment may have one or more related AEs, and each AE may have the same or a different severity level. Reference to a method capable of "altering an adverse event" refers to a regimen that reduces the incidence and/or severity of one or more AEs associated with the use of a different regimen.

As used herein, "administration interval" refers to the amount of time that elapses among multiple doses of a formulation disclosed herein administered to an subject. The administration interval may thus be indicated as a range.

As used herein, the term "administration frequency" refers to the frequency at which doses of a formulation disclosed herein are administered over a given time. The administration frequency may be indicated as the number of administrations per given time, e.g., once every week or once every two weeks. The term "flat dose" refers to a dose administered to a patient without considering the weight or the body surface area (BSA) of the patient. Thus, the flat dose is specified as the absolute amount of a medicament (e.g., an anti-PCSK9 antibody) rather than the mg/kg dose. For example, a 60-kg human and a 100-kg human will receive the same dose of an antibody (e.g., 240 mg of an anti-PCSK9 antibody).

The term "fixed dose" in reference to the composition disclosed herein means that two or more different antibodies in a single composition are present in the composition in a specific (fixed) ratio to each other. In certain embodiments, the fixed dose is based on the weight of the antibody (e.g., mg). In certain embodiments, the fixed dose is based on the concentration of the antibody (e.g., mg/mL). In certain embodiments, the ratio of a first antibody (mg) to a second antibody (mg) is at least about 1:1, about 1:2, about 1:3, about 1:4, about 1:5, about 1:6, about 1:7, about 1:8, about 1:9, about 1:10, about 1:15, about 1:20, about 1:30, about 1:40, about 1:50, about 1:60, about 1:70, about 1:80, about 1:90, about 1:100, about 1:120, about 1:140, about 1:160, about 1:180, about 1:200, about 200:1, about 180:1, about 160:1, about 140:1, about 120:1, about 100:1, about 90:1, about 80:1, about 70:1, about 60:1, about 50:1, about 40:1, about 30:1, about 20:1, about 15:1, about 10:1, about 9:1, about 8:1, about 7:1, about 6:1, about 5:1, about 4:1, about 3:1 or about 2:1. For example, a 3:1 ratio of the first antibody and the second antibody may refer to that a vial may contain about 240 mg of the first antibody and 80 mg of the second antibody, or about 3 mg/mL of the first antibody and 1 mg/mL of the second antibody.

A "patient" (sometimes denoted as a "subject") includes any human or non-human animal. The term "non-human animal" includes, but is not limited to, vertebrates such as non-human primates, sheep, dogs, and rodents such as mice, rats and guinea pigs. In certain embodiments, the patient is a human. The terms "subject" and "patient" are used interchangeably in certain contexts herein.

A "therapeutically effective amount" or "therapeutically effective dose" of a drug or therapeutic agent is any amount of a drug that, when used alone or in combination with another therapeutic agent, protects a patient from the onset of a disease or promotes disease regression as evidenced by reduction in the severity of disease symptoms, increase in the frequency and duration of disease symptom-free periods, or the prevention of damage or disability caused by the affliction of the disease. The ability of a therapeutic agent to promote disease regression can be evaluated using a variety of methods known to skilled practitioners, such as in a human patient in clinical trials, in an animal model system that predicts the efficacy for humans, or by determining the activity of the drug in an *in vitro* assay.

The terms "about", "approximately" or "substantially comprise" refers to a value or composition within an acceptable error range from the particular value or composition as determined by those of ordinary skill in the art, which will depend in part on how the value or composition is measured or determined, i.e., the limitations of the measurement system. For example, "about", "approximately" or "substantially comprise" may refer to being within 1 or more than 1 standard deviation as practiced in the art. Alternatively, "about" or "substantially comprise" may refer to a range that differs by up to 10% or 20% (i.e., ±10% or ±20%) from the parameter or value modified thereby. For example, about 3 mg may include any number between 2.7 mg and 3.3 mg (for 10%) or between 2.4 mg and 3.6 mg (for 20%). Furthermore, particularly with respect to biological systems or processes, the term can refer to up to an order of magnitude or up to at most 5 times the numerical value. When a particular value or composition is provided in the present application and claims, unless otherwise stated, the meaning of "about" or "substantially comprise" should be assumed to be within an acceptable error range from the particular value or composition.

As used herein, the term "about once every week", "about once every two weeks" or any other similar administration interval term refers to an approximation. "About once every week" may include once every 7±1 days, i.e., once every 6 days to 8 days. "About once every two weeks" may include once every 14±3 days, i.e., once every 11 days to 17 days. Similar approximations apply to, for example, about once every 3 weeks, about once every 4 weeks, about once every 5 weeks, about once every 6 weeks, and about once every 12 weeks. In certain embodiments, an administration interval of about once every 6 weeks or about once every 12 weeks means that the first dose may be administered on any day of the first week, and then the second dose may be administered on any day of the sixth or twelfth week, respectively. In other embodiments, an administration interval of about once every 6 weeks or about once every 12 weeks means that the first dose is administered on a particular day (e.g., Monday) of the first week and then the second dose is administered on the same day (e.g., Monday) of the sixth or twelfth week, respectively. Similar principles apply to phrases including but not limited to, "about once every 2 weeks", "about once every month", etc.

As used herein, unless otherwise indicated, any concentration range, percentage range, ratio range, or integer range shall be understood as including the value of any integer within the listed range and including, when appropriate, fractions thereof (such as one tenth and one hundredth of the integer).

Unless otherwise stated, "about" or "approximately" in present application means being within ±5%, preferably within ±2%, and more preferably within ±1% of the specified numerical range given. For example, a pH of about 5.5 means a pH of 5.5±5%, preferably a pH of 5.5±2%, and more preferably a pH of 5.5±1%.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutical formulation" or "pharmaceutical composition" refers to a mixture consisting of an active ingredient (e.g., an anti-PCSK9 antibody) disclosed herein and a pharmaceutically acceptable excipient. The pharmaceutical composition is intended to facilitate the administration of the active ingredient disclosed herein to a subject.

The term "single dose unit" means a single pharmaceutical dosage form, such as an ampoule, comprising the anti-PCSK9 antibody or the antigen-binding fragment thereof disclosed herein to be administered to a subject at time points of a regimen, preferably per kg body weight of the subject.

### Modes of Administration

The content below is not intended to limit the modes of administration of the medicament disclosed herein.

In one embodiment, the medicament disclosed herein can be formulated into a pharmaceutical composition which is suitable for a single dose or multiple doses.

The medicament disclosed herein is administered in various proper routes, including but not limited to, oral administration or parenteral administration (by intravenous, intramuscular, local or subcutaneous routes). In some embodiments, the medicament disclosed herein is administered by means of oral administration or injection, for example, intravenous injection or intraperitoneal injection.

Suitable dosage forms of the medicament disclosed herein include, but are not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, pulvis and dosage forms of sustained release formulations for oral or non-oral administration.

The pharmaceutical composition disclosed herein comprises a pharmaceutically acceptable carrier and/or excipient.

### BRIEF DESCRIPTION OF THE DRAWINGS

FIG. 1 illustrates the effect of MAB1 on mouse serum LDL-C.
FIG. 2 illustrates the effect of MAB1 on cynomolgus monkey serum LDL-C (s.c.: subcutaneous injection; i.v.: intravenous injection).
FIG. 3 illustrates the effect of MAB1 on the endocytosis of LDL by HepG2 cells.
FIG. 4 illustrates MAB1 relieving the inhibition of LDL endocytosis by wild-type PCSK9.
FIG. 5 illustrates MAB1 relieving the inhibition of LDL endocytosis by functionally enhanced mutant PCSK9 (PCSK9-D374Y).

### DETAILED DESCRIPTION

The present application is further described below with reference to specific examples, which are, however, only for illustration and do not limit the scope of the present application. Likewise, the present application is not limited to any particular preferred embodiment described herein. It should be understood by those skilled in the art that equivalent substitutions and corresponding modifications of the technical features of the present application still fall within the protection scope of the present application. Unless otherwise stated, the reagents used in the following examples are commercially available products, and the solutions can be prepared by conventional techniques in the art.

In the following examples of the present invention, the isotype control antibody was human anti-hen egg lysozyme IgG (anti-HEL; i.e., human IgG, abbreviated as hIgG), and its sequence was from Affinity Maturation Increases the Stability and Plasticity of the Fv Domain of Anti-Protein Antibodies (Acierno et al., J Mol Biol., 2007, 374(1):130-46, wherein the amino acid sequence of the heavy chain is set forth in SEQ ID NO: 11, and the amino acid sequence of the light chain is set forth in SEQ ID NO: 12). The isotype control antibody was prepared in the laboratory of Akeso Biopharma, Inc.

In the following examples of the present invention, C57BL/6 mice were purchased from Guangdong Medical Laboratory Animal Center.

### Example 1. Preparation of Humanized Anti-PCSK9 Antibody

### 1. Design of antibody

In order to prepare monoclonal antibody MAB1, the inventors designed a series of antibody sequences based on the known PCSK9 protein sequence (NP_777596.2) and three-dimensional crystal structure thereof. Through extensive screening and analyses, the antibody MAB1 that specifically binds to PCSK9 was finally obtained. The amino acid sequences of the heavy chain and the light chain variable regions of the monoclonal antibody and the encoding sequences thereof are set forth in SEQ ID NOs: 1-4 below.
The DNA sequence of the heavy chain VH of the designed MAB1 is as follows (369 bp): 
GAGGTGCAGCTGGTGGAGTCTGGAGGAGGCCTGGTGCAGCCCGGA
AGATCTCTGAGACTGAGTTGCGCCGCTTCAGGATTCACCTTTAGCTC
CTACAGCATGAACTGGGTGCGGCAGGCTCCTGGCAAGGGGCTGGAG
TGGGTCTCCGGAATCTCTAGTTCAAGCTCCTACATTAGCTATGCAGA
CTCCGTCCAGGGAAGGTTCACCATCTCTCGCGATAACGGCAAGAAC
AGCCTGTATCTGCAGATGAACAGCCTGCGAGCAGAGGACACAGCCC
TGTACTTCTGTGCCAGAGAATATGACTTCTGGTCCGCCTATTACGAC
GCCTTCGATGTCTGGGGACAGGGGACTATGGTCACTGTCTCAAGC
(SEQ ID NO: 1)
The protein sequence of the encoded VH is as follows (123 aa): 
EVQLVESGGGLVQPGRSLRLSCAASGFTFSSYSMNWVRQAPGKGLEW
VSGISSSSSYISYADSVQGRFTISRDNGKNSLYLQMNSLRAEDTALYFC
AREYDFWSAYYDAFDVWGQGTMVTVSS (SEQ ID NO: 2)
The DNA sequence of the light chain VL of MAB1 is as follows (333 bp): 
CAGAGCGAACTGACTCAGCCAAGAAGCGTCAGTGGATCACCTGGCC
AGAGCGTGACAATCTCCTGCACCGGCACAAGCAGGAACATTGGCGG
 
GGGAAATGACGTCCACTGGTACCAGCAGCATCCAGGGAAGGCCCCC
AAACTGCTGATCTCCGGAGTGATTGAGCGGAGCTCCGGCGTCCCCG
ATAGATTCAGCGGGTCCAAGTCTGGAAACACAGCTTCTCTGACTAT
CAGTGGCCTGCAGGCAGAGGACGAAGCCGATTACTATTGCCAGTCT
TTCGACGGCAGTCTGTCAGGGAGCGTGTTTGGCACTGGGACCGATG
TGACCGTCCTG (SEQ ID NO: 3)
The protein sequence of the encoded VL is as follows (111 aa):

QSELTQPRSVSGSPGQSVTISCTGTSRNIGGGNDVHWYQQHPGKAPKL
LISGVIERSSGVPDRFSGSKSGNTASLTISGLQAEDEADYYCQSFDGSLS
GSVFGTGTDVTVL (SEQ ID NO: 4)

The CDR sequences of the antibody are as follows:
HCDR1: GFTFSSYS (SEQ ID NO: 5);
HCDR2: ISSSSSYI (SEQ ID NO: 6);
HCDR3: EYDFWSAYYDAFDV (SEQ ID NO: 7);
LCDR1: SRNIGGGND (SEQ ID NO: 8);
LCDR2: GVI (SEQ ID NO: 9); and
LCDR3: QSFDGSLSGSV (SEQ ID NO: 10).

### 2. Expression and purification of antibody

The heavy chain cDNA sequence (coding sequence set forth in SEQ ID NO: 1; the constant region was Ig gamma-1 chain C region, ACCESSION: P01857) and the light chain cDNA sequence (coding sequence set forth in SEQ ID NO: 3; the constant region was Ig lambda-2 chain C regions; ACCESSION: P0CG05.1) of MAB1 were separately cloned into pUC57simple vectors (supplied by GenScript) and pUC57simple-MAB1H and pUC57simple-MAB1L plasmids were obtained, respectively. pUC57simple-MAB1H and pUC57simple-MAB1L plasmids were digested (HindIII&EcoRI), and the heavy and light chains isolated by electrophoresis were separately subcloned into pcDNA3.1 vectors, and recombinant plasmids were extracted to co-transfect 293F cells. After 7 days of cell culture, the culture medium was separated by centrifugation at high speed, and the supernatant was concentrated and loaded onto a HiTrap MabSelect SuRe column. The protein was eluted in one step with an elution buffer. The target sample was isolated and the buffer was exchanged into PBS.

### Example 2. Effect of MAB1 on Animal Serum Low-Density Lipoprotein Cholesterol LDL-C

### (1) Effect of MAB1 on mouse serum low-density lipoprotein cholesterol LDL-C

Serum was isolated from orbital blood sample collected from 32 C57BL/6 mice before administration. Low-density lipoprotein cholesterol (LDL-C) in mice was detected by a fully automated biochemical analyzer (Mindray, model BS-180). As shown in Table 1, the mice were divided into 4 groups of 8 mice, i.e., normal group, model group, MAB1 low dose group and MAB1 high dose group, and were treated according to the dosing regimen in Table 1. Serum was separated from blood samples collected before administration (D0), and after administration on D3, D7, D10, D14 and D18, and the low-density lipoprotein cholesterol content was detected.

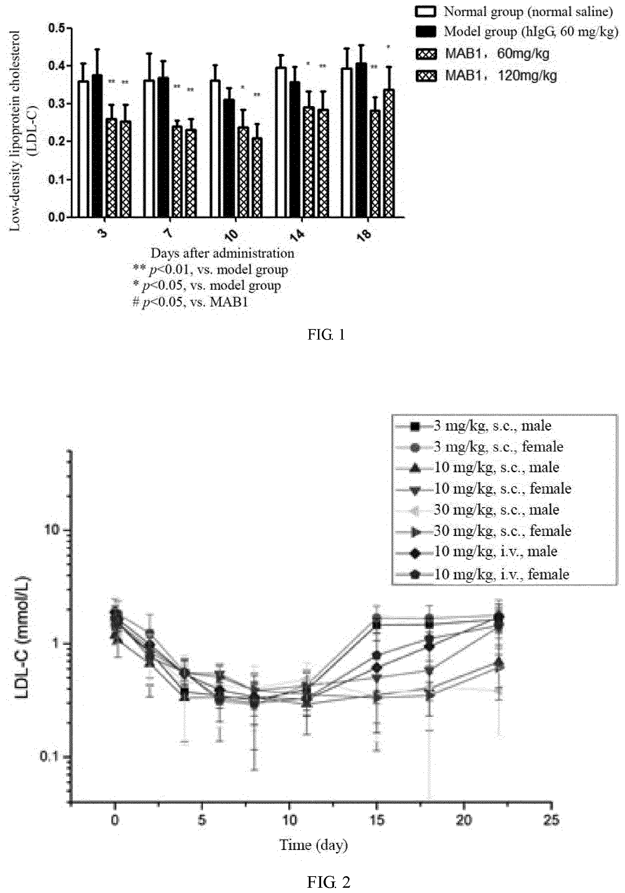

The results are shown in FIG. 1. The experiment results showed that compared with the model group, the mice serum LDL-C concentration was significantly reduced in the MAB1 low-dose 60 mg/kg group and the MAB1 high-dose 120 mg/kg group (P < 0.05 or P < 0.01).

**Table 1. Dosage and regimen of MAB 1 for mice**

| Group | Animal Number | Blood sampling scheme | Dosing regimen (including treatment, dosage, site, frequency, etc.) |
|---|---|---|---|
| Normal group | 8 | Serum was separated from blood samples collected before administration (D0), and after | Saline, s.c., DO |
| Model group | 8 | | hIgG, 60 mg/kg, s.c., DO |
| MAB1 60mg/kg | 8 | administration on D3, D7, D10, D14 and D18, and the low-density lipoprotein cholesterol content was detected. | MAB1, 60 mg/kg, s.c., DO |
| MAB1 120mg/kg | 8 | | MAB1, 120 mg/kg, s.c., DO |

| | | | |
|---|---|---|---|
| Note: the first dose was given on DO (day 0); s.c.: subcutaneous administration; | | | |

### (2) Effect of MAB 1 on cynomolgus monkey serum low-density lipoprotein cholesterol LDL-C

The experiment was conducted using 12 male and 12 female cynomolgus monkeys (general grade) purchased from Nanning Deheng Biotechnology Ltd. (animal production license no. SCXK(GUI)2011-0007; laboratory animal certificate no. 0005869). All procedures and activities involving use and welfare of the animal were approved by the Institutional Animal Care and Use Committee (IACUC) prior to the procedures. IACUC approval no.: ACU16-467.

As shown in Table 2, animals were randomized by gender to MAB1 low dose group, MAB1 moderate dose group, MAB1 high dose group, and MAB1 intravenous administration group using a computer program based on weight of the animals measured prior to dosing, and were treated according to the dosing regimen in Table 2. About 0.5 mL of blood was collected at fasting on DO before administration and at 4 h and on D2, D4, D6, D8, D11, D15, D18 and D22 after administration. The blood samples were transferred into centrifuge tubes and centrifuged for 10 minutes at 2-8 °C at 3000 g. The low-density lipoprotein cholesterol (LDL-C) was detected.

The results are shown in FIG. 2. Conclusions: MAB1 was administered to cynomolgus monkeys in a single subcutaneous injection at doses of 3, 10 and 30 mg/kg, and in a single intravenous injection at 10 mg/kg. The low-density lipoprotein cholesterol in the cynomolgus monkeys was significantly reduced after administration. The blood lipid level in the 3 mg/kg dose group basically returned to the baseline level in about 15 days after administration; the blood lipid level in the 10 mg/kg s.c. group and the i.v. administration groups also basically returned to the baseline in about 22 days after administration, and the blood lipid level in the 30 mg/kg dose group was still significantly lower than the baseline.

**Table 2. Dosage and regimen of MAB1 for cynomolgus monkeys**

| Group | Animal Number | Blood sampling scheme | Dosing regimen |
|---|---|---|---|
| Low dose group | 6 | About 0.5 mL of blood was collected at fasting on DO before administration and at 4 h and on D2, D4, D6, D8, D11, D15, D18 and D22 after administration. Serum was separated from the blood samples, and the low-density lipoprotein cholesterol content was detected. | MAB1, 3 mg/kg, s.c., DO |
| Moderate dose group | 6 | | MAB1, 10 mg/kg, s.c., DO |
| High dose group | 6 | | MAB1, 30 mg/kg, s.c., DO |
| Intravenous administrati on group | 6 | | MAB1, 10 mg/kg, i.v., DO |

| | | | |
|---|---|---|---|
| Note: the first dose was given on DO (day 0); s.c.: subcutaneous administration; i.v.: intravenous administration; the animals were randomized by gender into 4 groups each containing 3 males and 3 females using a computer program based on weight of the animals measured prior to dosing. | | | |

### Example 3. Effect of MAB1 on Endocytosis of LDL by HepG2 Cells

PCSK9 may inhibit the expression of LDLR on HepG2 cell surface by mediating endocytosis and degradation of LDLR. LDL can be bind to LDLR and can be endocytosed by the cell. BODIPY-labeled LDL (BODIPY-LDL, Life, cat. no.: L3483) can be used as a label to bind to LDLR and can be endocytosed by the cell. The fluorescence value of the intracellular BODIPY-LDL can be detected to reflect the LDLR-mediated endocytosis of LDL. If PCSK9 is present and binds to LDLR, the amount of LDLR on the cell surface is reduced, thus reducing the binding of BODIPY-LDL to LDLR and inhibiting the endocytosis of LDL; on the contrary, inhibition of PCSK9 enhances endocytosis of LDL by HepG2 cells. Therefore, the test system can measure the pharmacological activity of the anti-PCSK9 antibody by detecting the endocytic activity of the HepG2 cells on BODIPY-LDL.

Procedures: 50 µL of polylysine (Poly-L-lysine) solution was added into each well of a 96-well black bottom cell culture plate for coating. The plate is incubated in a 37 °C, 5% CO₂ incubator for 2 hours, and the coating solution was discarded. The plate was washed once with 100 µL of PBS and dried for later use. HepG2 cells were conventionally digested, resuspended in an assay medium (DMEM with 1% NEAA), counted and seeded at 2 × 10⁴ cells/well on the 96-well black-bottom cell culture plate in a volume of 80 µL/well. The cells were incubated in a 37 °C, 5% CO₂ incubator for 18 hours. PCSK9 was pre-diluted to 6000 nM with the assay medium (DMEM with 1% NEAA), and added to the plate at 10 µL/well. The antibody was diluted by 10 folds, and added at 10 µL/well. The mixture was incubated in a 37 °C, 5% CO₂ incubator for 6 hours. Thereafter, 10 µL of 0.1 mg/mL BODIPY-LDL solution was added to each well and the mixture was incubated in a 37 °C, 5% CO₂ incubator for 22 hours. The medium was discarded, and 200 µL of PBS was added to each well for washing. A formaldehyde solution was added to fix the cells and the cells were let stand at room temperature for 20 min. The formaldehyde solution was discarded and a PBS was added for washing. Finally, the PBS was discarded, and 100 µL of PBS was added into each well. The relative fluorescence unit of BODIPY was detected by a multi-label microplate tester at an exciting wavelength of 490 nm, an emitting wavelength of 520 nm and a cutoff of 515 nm.

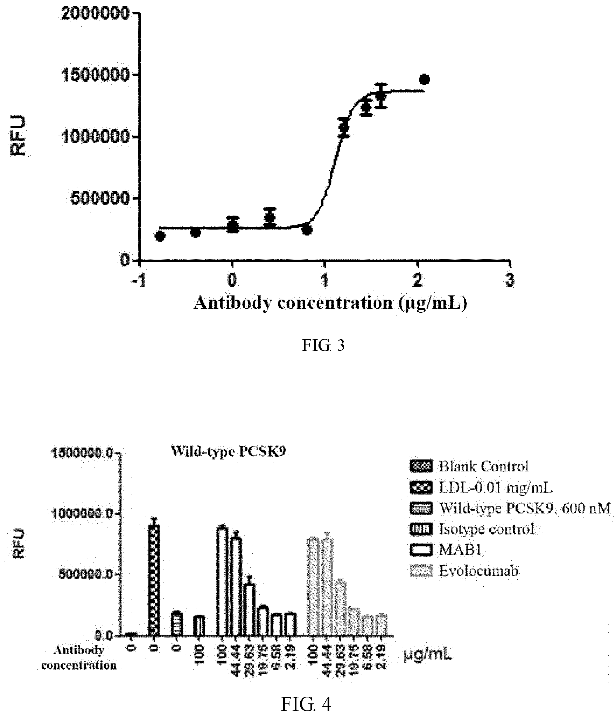

Results as shown in Table 3 and FIG. 3. The EC₅₀ value for MAB1 relieving the inhibition of LDL endocytosis in HepG2 cells by PCSK9 was 12.99 µg/mL.

Conclusions: MAB1 can inhibit the activity of PCSK9, thus elevating LDL endocytosis in HepG2 cells; the EC₅₀ of MAB1 is 12.99 µg/mL.

**Table 3. Assay of LDL endocytosis in HepG2 cells by MAB1**

| **MAB1** concentration (µg/mL) | RFU | Mean | | SEM |
|---|---|---|---|---|
| 120 | 1432819 | 1501195 | 1467007 | 34188 |
| 40 | 1421783 | 1233042 | 1327413 | 94370.5 |
| 28 | 1294536 | 1179255 | 1236896 | 57640.5 |
| 16 | 1145831 | 1002411 | 1074121 | 71710 |
| 6.4 | 267495 | 227003 | 247249 | 20246 |
| 2.56 | 412355 | 282301 | 347328 | 65027 |
| 1.024 | 341749 | 236159 | 288954 | 52795 |
| 0.4096 | 222579 | 229323 | 225951 | 3372 |
| 0.16384 | 203970 | 192901 | 198435.5 | 5534.5 |

### Example 4. MAB1 relieving inhibition of LDL endocytosis by wild-type and functionally enhanced mutant PCSK9

PCSK9 has multiple mutants, and is divided into two types, gain-of-function and loss-of-function according to the effect of the mutation on regulation of LDL-C level by PCSK9. Gain-of-function mutation enhances PCSK9's ability to up-regulate LDL-C, resulting in hypercholesterolemia, and mainly includes S127R, D129G, F216L, R218S, D374Y, N425S, R496W, H553R, E670G, etc. Among these, the D374Y (Asp374Tyr) mutation increases the affinity of PCSK9 for LDLR by 5 to 30 folds (Lagace TA et al., J. Clin. Invest., 2006, 116(11):2995-3005; Cunningham D et al., Nat. Struct. Mol. Biol., 2007, 14(5):413-419; Fisher TS et al., J. Biol. Chem., 2007, 282(28): 20502-20512). Taking the D374Y (Asp374Tyr) mutant protein as an example, this experiment investigated the activity of MAB1 on inhibiting LDLR endocytosis mediated by functionally enhanced PCSK9 mutant protein. The results suggest that MAB1 can effectively relieve the inhibition of LDL endocytosis by gain-of-function PCSK9 protein.

Materials: HepG2 (purchased from Chinese Academy of Sciences, P14); DMEM (Gibco, cat. no. 11995040); FBS (Excell Bio, cat. no. FSP500); MEM NEAA (100×) (Gibco, cat. no. 11140-050); POLY-L-LYSINE (SIGMA, cat. no. P4832); BODIPY-LDL (Life, cat. no. L3483); PCSK9-his (prepared by Akeso Biopharma); PCSK9(D374Y)-his (Acrobio, cat. no. PCY-H5225); evolocumab (purchased from AMGEN).

Procedures: A 96-well black bottom cell culture plate was coated with 50 µL of 0.01% poly-L-lysine. The plate is incubated in an 37 °C, 5% CO₂ incubator for 2 hours, and the coating solution was discarded. The plate was washed once with 100 µL of PBS and dried for later use. For HepG2 cells in log phase, the growth medium (12% FBS, DMEM with 1% NEAA) was discarded. The cells were resuspended in an assay medium (DMEM with 1% NEAA) after trypsinization and counted. The cells were seed at 20,000 cells/well on the 96-well black cell culture plates in a volume of 80 µL/well, and incubated at 37 °C/5% CO₂ overnight. The next day, PCSK9 and the antibody (with final concentrations shown in the figure) were diluted in an assay medium and added to a 96-well plate in a final volume of 100 µL. The cell culture plate was incubated at 37 °C/5% CO₂ for 21 h. The culture plate was taken out, and 10 µL of 0.1 mg/mL BODIPY-labeled LDL solution was added. The mixture was cultured at 37 °C/5% CO₂ for 9 h. The medium was discarded, and 200 µL of PBS was added to each well for washing. 100 µL of PBS was added into each well. The relative fluorescence unit (RFU) of the plate was detected by a multi-label microplate tester at an exciting wavelength of 485 nm and an emitting wavelength of 535 nm.

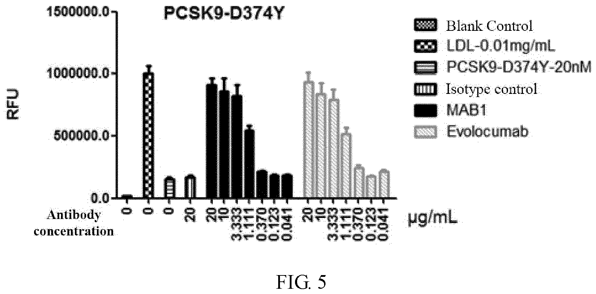

Results: As shown in Table 4, FIG. 4 and FIG. 5, MAB1 can effectively bind to wild-type PCSK9 and functionally enhanced mutant PCSK9 (PCSK9-D374Y), and effectively block PCSK9 from inhibiting LDL endocytosis, which is equivalent to the activity of the commercially available drug evolocumab with the same target.

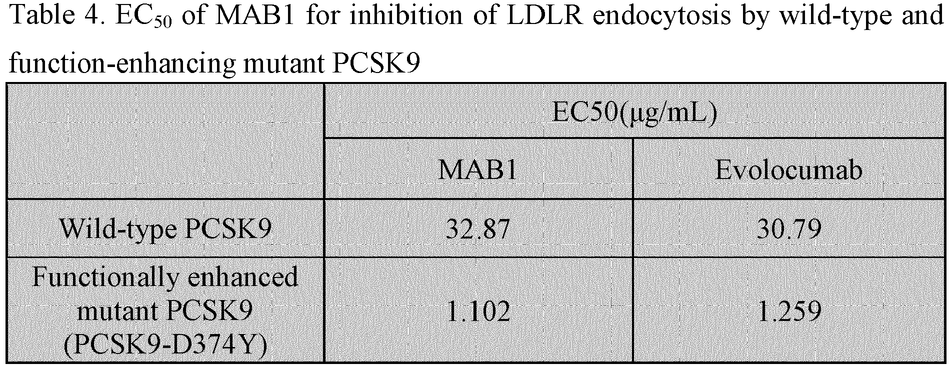

### Example 4. MAB1 relieving inhibition of mutant LDLR-mediated LDL endocytosis by wild-type PCSK9

The binding of PCSK9 protein to LDLR regulates LDL-C metabolism. The LDLR gene mutations are of a great number, and can be divided into the following according to LDLR protein synthesis and function: 1) LDLR non-expression mutations, including LDLR allele non-expression (i.e., LDLR is not expressed by the cells), for example, promoter sequence mutation, nonsense mutation, frameshift mutation, and splicing mutation , and LDLR transport defects i.e. defective LDLR cannot be transported from endoplasmic reticulum to Golgi apparatus after being synthesized and then is accumulated and decomposed in cells; 2) binding-deficient gene mutation, i.e., the abnormal LDLR encoded by the mutant gene can be transported and expressed on the cell surface, but has reduced or eliminated activity for binding LDL-C; 3) internalization-deficient gene mutation, i.e., a receptor that binds to LDL but cannot be endocytosed accumulates to cover caveolae due to a mutation in the codon encoding the NPVY sequence; 4) recycling-deficient gene mutations, i.e., the receptor is able to bind to LDL-C and mediate endocytosis but will be digested simultaneously with LDL-C in the lysosome, leading to the inability of LDLR to recycle to the cell surface. By taking the wild-type LDLR (with amino acid D at position 227 corresponding to the following mutant LDLR (D227E)) and a mutant LDLR (D227E; https://www.ncbi.nlm.nih.gov/clinvar/variation/3690/) as controls, this experiment evaluated the LDL-endocytosis activity of different mutant LDLRs, the inhibition of different mutant LDLR on LDL endocytosis by PCSK9, and whether inhibition of LDL endocytosis by PCSK9 was effectively relieved by MAB1.

Procedures: 293T cells were plated by conventional digestion, and seeded on 6-well plates at 5 × 10⁵ cells/well. 2 mL of DMEM + 10% FBS + 1% bispecific antibody (Penicillin Streptomyces, Gibco, cat. no. 15140-122) was added, and the cells were incubated overnight in an incubator. The medium was exchanged 2 h before transfection into 1 mL of Opti-MEM serum-free medium. The target plasmid DNA (constructed by Akeso Biopharma, Inc., see Table 5, based on wild-type LDLR, using the amino acid encoded by the initiation codon as position 1, and after performing site-specific mutagenesis of the corresponding position according to Table 5, the DNA was inserted into the lentiviral plasmid pCDH-CMV-MCS-EFl-puro purchased from YouBio to give the plasmids in Table 5) and PEI (Polyscience, cat. no.: 23966) were separately diluted with Opti-MEM, with the target plasmids and the control plasmids (constructed by Akeso Biopharma, Inc., see Table 5) each of 2 µg and PEI of 10 µg (5 µg of PEI separate transfection). The diluted DNA and PEI were mixed well and incubated for 15 min, and slowly and dropwise added into 6-well plates, gently mixed, and incubated in an incubator overnight. The medium in the 6-well plates was exchanged to 2 mL DMEM + 10% FBS, and the cells were incubated in the incubator for further incubation. After 48 h of transfection, the cells were collected by digestion with 0.05% pancreatin-EDTA and centrifugation, and counted. A portion of the cells was added to a reporter assay plate at 2 × 10⁵ cells/well, and 100 µL of DMEM + 10% FBS, PBS and IL-6 (Akeso Biopharma, lot no.: 20150421) (50 ng/mL) were added. The cells were incubated overnight, and 50 µL of substrate (Bright-Glo^{™} luciferase assay system, Promega, lot: E2620) was added to read the RLU. Another portion of the cells was used for LDL endocytosis (FACS) assay using 200 µL of Opti-MEM + 1% NEAA. The cells were added at 2 × 10⁵ cells/well, and co-incubated with PCSK-9, hIgG1 (Akeso Biopharma, lot no.: 20170424) and MAB1 at final concentrations of 600 nM, 50 µg/mL and 50 µg/mL, respectively, where the cells were pre-incubated with PCSK-9 and antibody before the compound was added for a further incubation overnight. Corresponding LDL (BODIPY-LDL, Life, cat. no.: L3483; final concentration: 0.001 mg/mL) was added according to the experimental design, and the mixture was well mixed and incubated for 4 h;

The cells were collected, transferred to a 96-well plate with a pointed bottom, and centrifuged for 5 min at 350× g. The supernatant was discarded. The cell precipitate was resuspended in 200 µL of 1% PBSA and centrifuged for 5 min at 350× g before the supernatant was discarded. The washing was repeated once. The cell precipitate was resuspended in 200 µL of 1% PBSA and transferred to a flow cytometry tube for assay.

The results are shown in Table 5. Taking wild-type LDLR as an example, the LDL endocytosis rate was 11.50%; after PCSK9 was added, the endocytosis rate was 7.23%, suggesting that PCSK9 blocked the LDL endocytosis by LDLR; when MAB1 was added on the basis of LDL and PCSK9, the endocytosis rate was 8.53%, suggesting that MAB1 can effectively relieve the PCSK9-mediated inhibition of LDL endocytosis. The mutation sequence information in the tables was obtained from the literature and associated online databases (Liang L et al., Scientific Reports, 2015; 5(1):17272.; https://www.ncbi.nlm.nih.gov/clinvar/).

**Table 5. Endocytosis rates % of BODIPY-LDL by wild-type LDLR and different mutant LDLRs**

| **Mutation site** | **Plasmid** | **LDL endocytosis rate%** | | | | **PCSK9 Inhibition %** |
|---|---|---|---|---|---|---|
| | | **LDL** | **LDL+ PCSK9** | **LDL+PCSK9 +hIgG1** | **LDL+PCSK9 + MAB1** | |
| hLDLRFL (wild type) | pCDH -hLDLRFL-puro | 11.50 | 7.23 | 6.27 | 8.53 | 37.13 |
| Asp227Glu (hLDLR(D227E)) | pCDH-hLDLR(D227E) -puro | 11.30 | 6.72 | 7.63 | 11.60 | 40.53 |
| Glu179Lys | pCDH-hLDLRmt2-puro | 9.30 | 2.04 | 2.21 | 7.11 | 78.06 |
| His583Asp | pCDH-hLDLRmt3 -puro | 7.56 | 2.88 | 2.96 | 6.06 | 61.90 |
| Asp172Asn | pCDH-hLDLRmt4.1-puro | 11.30 | 4.71 | 4.22 | 10.90 | 58.32 |
| Leu575Phe | pCDH-hLDLRmt4.2-puro | 7.18 | 5.01 | 5.08 | 5.93 | 30.22 |
| Cys255Arg | pCDH-hLDLRmt6.2-puro | 11.90 | 7.08 | 6.75 | 9.77 | 40.50 |
| Gly636Val | pCDH -hLDLR020-puro | 9.79 | 4.72 | 4.83 | 8.24 | 51.79 |
| Gly98Ser | pCDH-hLDLR059.1-puro | 11.60 | 7.11 | 7.47 | 9.96 | 38.71 |
| Asp94Asn | pCDH-hLDLR0123.1-puro | 11.60 | 3.98 | 3.34 | 7.42 | 65.69 |
| Ala459Thr | pCDH-hLDLRO 123.2-puro | 9.69 | 6.74 | 7.40 | 8.90 | 30.44 |
| His583Tyr | pCDH-hLDLR0160-puro | 9.04 | 6.36 | 6.96 | 8.17 | 29.65 |

The results showed that when the mutant LDLRs have LDL-endocytosis activity and PCSK9 can inhibit the LDL endocytosis, the addition of MAB1 can significantly increase the LDL endocytosis. That is, MAB 1 can effectively relieve PCSK9-mediated inhibition of LDL endocytosis.

## Claims

1. An anti-PCSK9 antibody or an antigen-binding fragment thereof for treating hypercholesterolemia.

2. The anti-PCSK9 antibody or the antigen-binding fragment thereof according to claim 1, wherein the anti-PCSK9 antibody comprises:
an HCDR1: GFTFSSYS (SEQ ID NO: 5);
an HCDR2: ISSSSSYI (SEQ ID NO: 6);
an HCDR3: EYDFWSAYYDAFDV (SEQ ID NO: 7);
an LCDR1: SRNIGGGND (SEQ ID NO: 8);
an LCDR2: GVI (SEQ ID NO: 9); and
an LCDR3: QSFDGSLSGSV (SEQ ID NO: 10).

3. The anti-PCSK9 antibody or the antigen-binding fragment thereof according to claim 1, wherein the anti-PCSK9 antibody comprises: a heavy chain variable region comprising an amino acid sequence set forth in SEQ ID NO: 2 or an amino acid sequence having at least 80% homology, preferably at least 85% homology, more preferably at least 90%, 91%, 92%, 93%, 94%, 95%, 96%, 97%, 98% or 99% homology thereto; and
a light chain variable region comprising an amino acid sequence set forth in SEQ ID NO: 4 or an amino acid sequence having at least 80% homology, preferably at least 85% homology, more preferably at least 90%, 91%, 92%, 93%, 94%, 95%, 96%, 97%, 98% or 99% homology thereto.

4. The anti-PCSK9 antibody or the antigen-binding fragment thereof according to claim 1, wherein the antibody is a monoclonal antibody, preferably a humanized antibody.

5. A pharmaceutical composition, preferably for treating hypercholesterolemia, comprising: the anti-PCSK9 antibody or the antigen-binding fragment thereof according to any of claims 1-4, and a pharmaceutically acceptable carrier.

6. A kit, preferably for treating hypercholesterolemia, comprising the anti-PCSK9 antibody or the antigen-binding fragment thereof according to any of claims 1-4, wherein preferably, the kit further comprises a statin (preferably a 3-hydroxy-3-methylglutaryl coenzyme A (HMG-CoA) reductase inhibitor, more preferably selected from lovastatin, pravastatin, simvastatin, fluvastatin, atorvastatin, rosuvastatin and pitavastatin), and/or ezetimibe.

7. The kit according to claim 6, wherein the anti-PCSK9 antibody is in a form suitable for a parenteral or non-parenteral route of administration.

8. A method for treating hypercholesterolemia, comprising administering (e.g., parenterally or non-parenterally) to a subject in need a therapeutically effective amount of the anti-PCSK9 antibody or the antigen-binding fragment thereof according to any of claims 1-4, wherein preferably, a statin is co-administered to the subject, and more preferably, a statin and ezetimibe are co-administered to the subject.

9. The method according to claim 8, wherein the anti-PCSK9 antibody or the antigen-binding fragment thereof, the statin, and/or ezetimibe are administered simultaneously or separately.

10. The method according to claim 8, wherein the therapeutically effective amount is 0.1 mg/kg or greater, preferably 0.1-60 mg/kg, based on the body weight of the patient.

11. The anti-PCSK9 antibody or the antigen-binding fragment thereof according to any of claims 1-4, the pharmaceutical composition according to claim 5, the kit according to any of claims 6-7, or the method according to any of claims 8-9, wherein the hypercholesterolemia is selected from the group consisting of:
(i) homozygous familial hypercholesterolemia, wherein more preferably the hypercholesterolemia is caused by 1 (pair of) or more (pairs of) allelic or non-allelic gene mutations in LDLR, apolipoprotein B (ApoB), PCSK9 or low-density lipoprotein receptor adaptor protein 1 (LDLRAP1) genes, preferably the allelic gene mutation including LDLR dysfunctional mutation, ApoB gene mutation affecting the binding of LDL to LDLR, functional mutation resulting in increased affinity of PCSK9 for LDLR, and LDLRAP1 dysfunctional mutation resulting in decreased LDL internalization activity, and/or preferably the hypercholesterolemia (more preferably HoFH) exhibits LDL-C > 13 mmol/L (500 mg/dL) when previously untreated or LDL-C ≥ 8 mmol/L (300 mg/dL) when previously treated (with a high dose/maximum tolerated dose of a statin in combination with ezetimibe), combined with skin or tendon xanthoma before age of 10 or both parents carrying 1 or more pairs of allelic gene mutations in LDLR, ApoB, PCSK9 or LDLRAP1 genes;
(ii) heterozygous familial hypercholesterolemia, wherein preferably the heterozygous familial hypercholesterolemia is caused by 1 or more unpaired allelic gene mutations in LDLR, apolipoprotein B (ApoB), PCSK9 or low-density lipoprotein receptor adaptor protein 1 (LDLRAP1) genes, and more preferably the allelic gene mutation includes LDLR dysfunctional mutation, ApoB gene mutation affecting the binding of LDL to LDLR, functional mutation resulting in increased affinity of PCSK9 for LDLR, and LDLRAP1 dysfunctional mutation resulting in decreased LDL internalization activity; and
(iii) hypercholesterolemia in a patient with extremely high or high risk of cardiovascular disease, wherein preferably the hypercholesterolemia in the patient with extremely high or high risk of cardiovascular disease refers to simultaneously satisfying the following condition 1 and condition 2, wherein the condition 1 is satisfying any of a) to h): a) a history of coronary artery disease (e.g. acute coronary syndrome, stable coronary heart disease, post-revascularization, ischemic cardiomyopathy), b) a history of ischemic stroke (excluding lacunar infarction), c) transient ischemic attack, d) peripheral atherosclerosis (e.g. carotid atherosclerosis, post-carotid endarterectomy, post-carotid stent placement, post-revascularization, lower limb atherosclerotic disease, post-lower limb artery revascularization), e) LDL-C ≥ 4.9 mmol/L (190 mg/dL) or TC ≥ 7.2 mmol/L, f) type 2 diabetes patients aged 40 years and above with 1.8 mmol/L (70 mg/dL) ≤ LDL-C < 4.9 mmol/L (190 mg/dL), g) chronic renal disease in stage 3 (i.e., with measured and calculated glomerular filtration rate 30 mL/min/1.73 m² ≤ eGFR < 60 mL/min/1.73 m²), h) at least 3 of the following risk factors: male aged ≥ 45 and female aged ≥ 55; hypertension; smoking; a family history of CVD (for first-degree relatives: male aged < 55 and female aged < 65); HDL cholesterol < 40 mg/dL; obesity (BMI ≥ 28 kg/m²); and the condition 2 is satisfying either a) or b): after 4 weeks of stable basal lipid-lowering agent treatment: a) LDL-C level > 1.8 mmol/L (70 mg/dL) for patients with extremely high risk, or b) LDL-C level > 2.6 mmol/L (100 mg/dL) for patients with high risk.

12. A method for treating a patient with hypercholesterolemia, comprising: (i) genotyping the patient, (ii) measuring LDL-C level in a sample (preferably serum, plasma or whole blood) from the patient, and (iii) administering to the patient, when the patient is identified with homozygous or heterozygous familial hypercholesterolemia, a therapeutically effective amount of an anti-PCSK9 antibody or an antigen-binding fragment thereof, preferably in combination with a statin, more preferably in combination with a statin and ezetimibe, wherein preferably the therapeutically effective amount is 0.1 mg/kg or higher, preferably 0.1-60 mg/kg, based on the weight of the patient.

13. The method according to claim 12, wherein the homozygous or heterozygous familial hypercholesterolemia is as defined in claim 11.

14. The method according to any of claims 12-13, wherein if the patient is identified with heterozygous familial hypercholesterolemia, the patient has a serum LDL-C level ≥ 4.7 mmol/L (180 mg/dL) when previously untreated with a lipid regulating drug, or,
if the patient is identified with heterozygous familial hypercholesterolemia, the patient satisfies at least 1 of the following a) and b) simultaneously: a) a patient having skin or tendon xanthoma or a patient aged < 45 years with arcus lipoides corneae, and b) having a first-degree relative with FH or early adult atherosclerotic cardiovascular disease (ASCVD), particularly coronary heart disease.

15. The method according to any of claims 8-12, wherein the anti-PCSK9 antibody or the antigen-binding fragment thereof is administered in a cycle of 2 weeks (14 days) or 4 weeks (28 days), and preferably, subcutaneously administered on the first day (D1) of each cycle.

16. Use of the anti-PCSK9 antibody or the antigen-binding fragment thereof according to any of claims 1-4 in preparing a pharmaceutical formulation (or a pharmaceutical composition) for reducing the risk of attack and death of cardiovascular disease in a patient with hypercholesterolemia at extremely high or high risk of cardiovascular disease.

17. A method for reducing blood cholesterol in a patient with hyperlipidemic cardiovascular disease (more preferably a patient with extremely high/high risk of cardiovascular disease) or for reducing a risk of cardiovascular disease attack in a patient with hyperlipidemic cardiovascular disease, comprising:
administering (e.g., parenterally or non-parenterally) to the patient a therapeutically effective amount of the anti-PCSK9 antibody or the antigen-binding fragment thereof according to any of claims 1-4, preferably in combination with a statin, more preferably in combination with a statin and ezetimibe, wherein more preferably the statin is selected from lovastatin, pravastatin, simvastatin, fluvastatin, atorvastatin, rosuvastatin and pitavastatin or any combination thereof.

18. The method according to claim 17, wherein the anti-PCSK9 antibody is administered in a cycle of 2 weeks (14 days) or 4 weeks (28 days), and preferably, subcutaneously administered on the first day (D1) of each cycle.
